# EUROPEAN PATENT APPLICATION

(11) **EP 4 654 209 A1**
(43) Date of publication of application: **26.11.2025**
(21) Application number: 24744555.4
(22) Date of filing: 11.01.2024
(51) Int. Cl.: G16H 10/60

(54) **INFORMATION EXCHANGE METHOD, PROGRAM, AND INFORMATION SYSTEM**

(30) Priority: 17.01.2023 JP 2023005215
(71) Applicant: SYSMEX CORPORATION, Kobe-shi Hyogo 651-0073 (JP)
(72) Inventor: OKADA, Seiki, Kobe-shi, Hyogo 651-0088 (JP); TANOSHIMA, Eiji, Kobe-shi, Hyogo 651-0088 (JP); YOSHIMURA, Hideki, Kobe-shi, Hyogo 651-0088 (JP); MATSUZAKI, Hazuki, Kobe-shi, Hyogo 651-0088 (JP); TANAKA, Tetsuhiro, Kobe-shi, Hyogo 651-0088 (JP)
(74) Representative: Hoffmann Eitle
(86) International application number: PCT/JP2024/000376
(87) International publication number: WO 2024/154632

(57) **Abstract**

It is made easy for a plurality of health professionals to flexibly cooperate according to the condition of a patient. An information exchange method for exchanging information about a patient among a plurality of health professionals by an information system (1) includes: receiving a group creation request based on at least one of patient identification information for identifying the patient and specialty attribute information about a specialty, in medicine, that is necessary for providing medicine to the patient; searching for a health professional satisfying the group creation request; creating, according to a result of the searching, a group including a health professional having made the group creation request and at least one of a health professional in charge of the patient and a health professional having the specialty, in medicine, that is necessary for providing medicine to the patient; and transmitting information about the patient transmitted from a terminal (3) of one of the health professionals in the created group, the information being transmitted to a terminal (3) of another one of the health professionals in the group.

## Description

### TECHNICAL FIELD

The present invention relates to a method, a program, and an information system for exchanging information about a patient among health professionals.

### BACKGROUND ART

Patent Literature 1 describes a home care assistance system in which pieces of information about a patient recuperating at home are collectively managed via a cloud server and in which a plurality of persons concerned mutually use the collectively managed pieces of information. In this system, a patient and persons who perform medical care on the patient are registered in the system as team members of one team by a system manager. The team members include the patient, the family of the patient, a doctor, a nurse, a physical therapist, a home caregiver, and the like. In this system, a communication terminal possessed by each of the team members and the cloud server are connected, and a condition (a facial expression, a blood pressure, a physical condition, or the like) of the patient, medical care (an instruction, a massage, medication, or the like) performed on the patient according to the condition, and the like are registered, as care records, from the communication terminal of any of the team members to a storage of the cloud server so that each of the team members views the registered information by using the corresponding communication terminal. In addition, in this system, when one of the team members registers or views a care record, the team member can register a communication such as a question or a comment directed to another one of the team members, and each of the team members can view the registered communication by using the corresponding communication terminal, in a screen corresponding to the care record.

### CITATION LIST

### [PATENT LITERATURE]

[PTL 1] Japanese Laid-Open Patent Publication No. 2016-91226

### SUMMARY OF THE INVENTION

### PROBLEMS TO BE SOLVED BY THE INVENTION

In a medical workplace, a plurality of health professionals are required to be able to flexibly cooperate according to the condition of a patient.

Increase in medical level and medical expertise have been progressing, and, in the case of performing advanced nursing such as chronic disease care or end-of-life care, expert knowledge and skills corresponding to the individual disease are required. Considering this, in a case where, for example, it is decided to perform home nursing on a patient who was admitted to a hospital, it is desirable that a plurality of health professionals such as a home-visiting nurse dispatched from a home-visiting nursing station and a specialized nurse who works at the hospital and who has expert knowledge and skills about nursing of the patient can cooperate to care for the patient. In particular, a plurality of types of nursing are sometimes necessary for one patient depending on the type or the degree of progress of the disease, and thus, even in the case of nursing the same patient, a health professional having a specialty corresponding to what the nursing involves and a health professional in charge of the nursing are required to be able to flexibly cooperate.

However, in the system described in the above Patent Literature 1, the system manager needs to register team members in advance. In a case where this system is used to perform, for example, home nursing requiring a high level of specialty such as the nursing described above, it is difficult for the system manager to register team members corresponding to the patient after sufficiently ascertaining details such as: a specialty of each of a plurality of health professionals belonging to different medical institutions such as a hospital and a home-visiting nursing station; a patient of whom the health professional is in charge (hereinafter, referred to as "assigned patient"); and the name of the health professional.

An object of the present invention is to provide a method, a program, and an information system that make it easy for a plurality of health professionals to flexibly cooperate according to the condition of a patient and that allow information about the patient to be exchanged among the health professionals.

### SOLUTION TO THE PROBLEMS

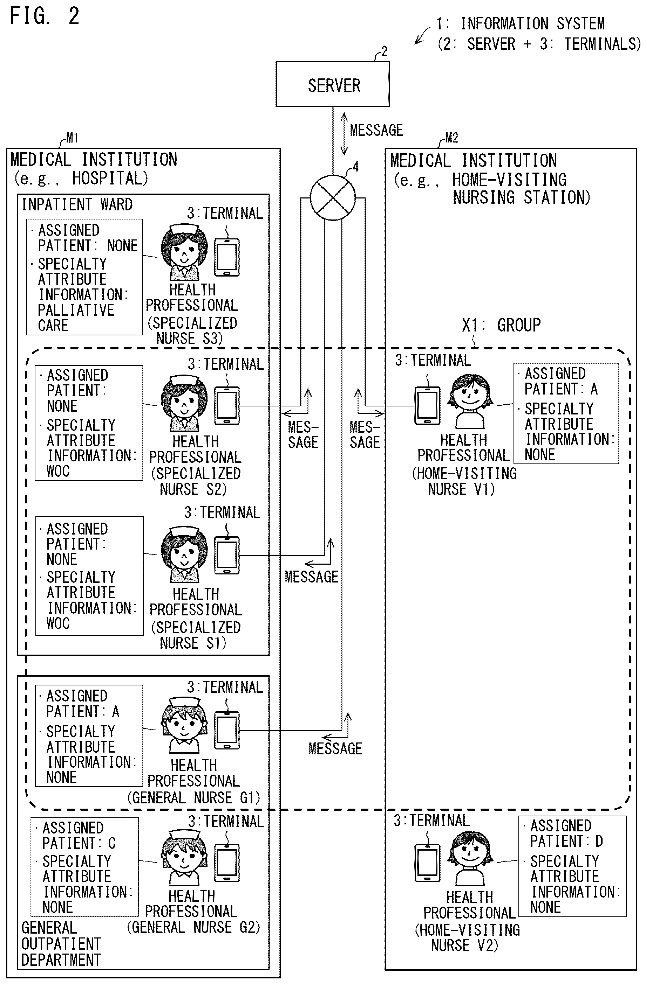

An information exchange method of the present invention is, as shown in FIG. 2, an information exchange method for exchanging information about a patient among a plurality of health professionals by an information system (1), the information exchange method including: receiving a group creation request based on at least one of patient identification information for identifying the patient and specialty attribute information about a specialty, in medicine, that is necessary for providing medicine to the patient; searching for a health professional satisfying the group creation request; creating, according to a result of the searching, a group including a health professional having made the group creation request and at least one of a health professional in charge of the patient and a health professional having the specialty, in medicine, that is necessary for providing medicine to the patient; and transmitting information about the patient transmitted from a terminal (3) of one of the health professionals in the created group, the information being transmitted to a terminal (3) of another one of the health professionals in the group.

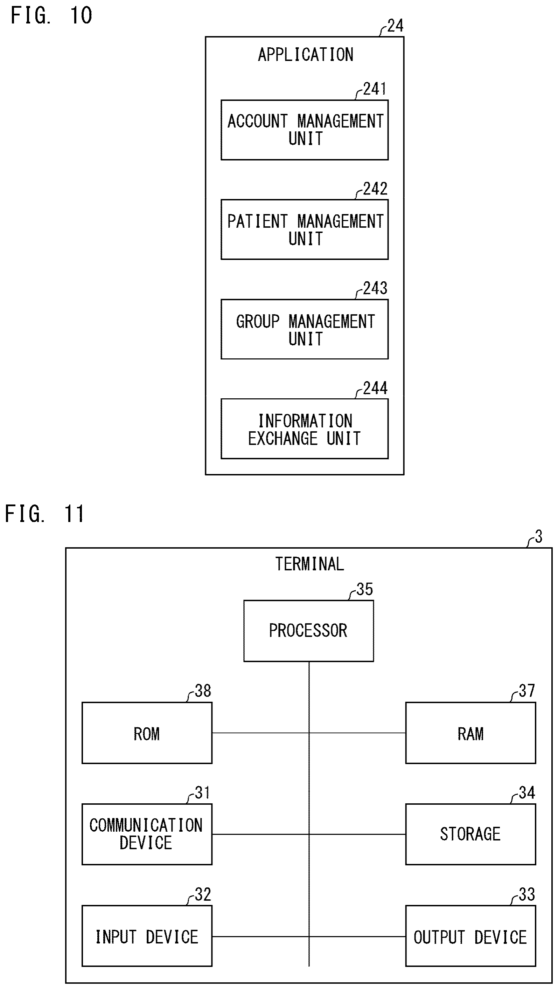

A program of the present invention is, as shown in FIG. 2 and FIG. 10, a program for causing a computer to function as an information processing device (2) included in an information system configured to exchange information about a patient among a plurality of health professionals, the information processing device (2) being configured to: receive a group creation request based on at least one of patient identification information for identifying the patient and specialty attribute information about a specialty, in medicine, that is necessary for providing medicine to the patient; search for a health professional satisfying the group creation request; create, according to a result of the searching, a group including a health professional having made the group creation request and at least one of a health professional in charge of the patient and a health professional having the specialty, in medicine, that is necessary for providing medicine to the patient; and transmit information about the patient transmitted from a terminal (3) of one of the health professionals in the created group, the information being transmitted to a terminal (3) of another one of the health professionals in the group.

An information system of the present invention is, as shown in FIG. 2, an information system (1) configured to exchange information about a patient among a plurality of health professionals, the information system (1) including: an information processing device (2); and a terminal (3) of each of the health professionals, wherein the information processing device (2) receives, from the terminal (3) of any of the health professionals, a group creation request based on at least one of patient identification information for identifying the patient and specialty attribute information about a specialty, in medicine, that is necessary for providing medicine to the patient, searches for a health professional satisfying the group creation request, creates, according to a result of the searching, a group including a health professional having made the group creation request and at least one of a health professional in charge of the patient and a health professional having the specialty, in medicine, that is necessary for providing medicine to the patient, and transmits information about the patient transmitted from the terminal (3) of one of the health professionals in the created group, the information being transmitted to the terminal (3) of another one of the health professionals in the group.

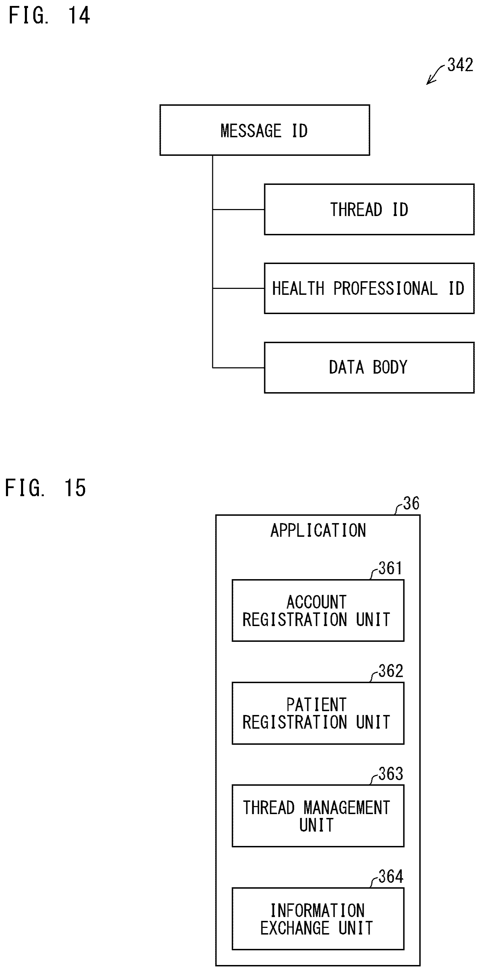

A program of the present invention is, as shown in FIG. 2 and FIG. 15, a program for causing a computer to function as a terminal (3) of each of a plurality of health professionals utilizing an information system (1) configured to exchange information about a patient among the health professionals, the terminal (3) being configured to: transmit, to an information processing device included in the information system, a group creation request based on at least one of patient identification information for identifying the patient and specialty attribute information about a specialty, in medicine, that is necessary for providing medicine to the patient; accept input of information about the patient; and transmit and receive the information about the patient among the terminals of health professionals in a group created on the basis of the group creation request.

### ADVANTAGEOUS EFFECTS OF THE INVENTION

The present invention makes it possible to provide a method, a program, and an information system that make it easy for a plurality of health professionals to flexibly cooperate according to the condition of a patient and that allow information about the patient to be exchanged among the health professionals.

### BRIEF DESCRIPTION OF THE DRAWINGS

FIG. 1 shows examples of nursing performed on a patient by health professionals;
FIG. 2 shows a configuration example of, and an example of group creation in, an information system according to an embodiment;
FIG. 3 shows an example of the group creation;
FIG. 4 shows an example of a thread screen;
FIG. 5 shows a configuration example of a server;
FIG. 6 shows a configuration example of a storage of the server;
FIG. 7 shows an example of a data structure of an account information database;
FIG. 8 shows an example of a data structure of a patient information database;
FIG. 9 shows an example of a data structure of a group information database;
FIG. 10 shows a configuration example of an application that operates in the server;
FIG. 11 shows a configuration example of a terminal;
FIG. 12 shows a configuration example of a storage of the terminal;
FIG. 13 shows an example of a data structure of a thread information database;
FIG. 14 shows an example of a data structure of a message information database;
FIG. 15 shows a configuration example of an application that operates in the terminal;
FIG. 16 shows an example of an account registration screen;
FIG. 17 shows an example of a patient registration screen;
FIG. 18 shows an example of a creation request screen;
FIG. 19 shows an example of a thread selection screen;
FIG. 20 shows flowcharts indicating an example of the flow of a process for creating a group and a thread;
FIG. 21 shows flowcharts indicating an example of the flow of an information exchange process;
FIG. 22 shows an example of a thread screen according to a modification;
FIG. 23 shows an example of a video call screen;
FIG. 24 shows a configuration example of a storage of a server according to a modification;
FIG. 25 shows an example of a data structure of a video call reservation information database;
FIG. 26 shows a configuration example of an application that operates in the server according to the modification;
FIG. 27 shows an example of a thread screen according to the modification;
FIG. 28 shows an example of a video call reservation screen;
FIG. 29 shows an example of a confirmation screen for a video call reservation;
FIG. 30 shows an example of a video call reservation search screen;
FIG. 31 shows a configuration example of an application that operates in a terminal according to a modification;
FIG. 32 shows an example of a thread screen according to the modification;
FIG. 33 shows an example of a report creation screen; and
FIG. 34 shows an example of a report.

### MODE FOR CARRYING OUT THE INVENTION

### [Overview of Information System 1]

An information system 1 according to the present embodiment is a communication system for exchanging information about a patient among a plurality of health professionals in a group including the health professionals. The information system 1 has a main feature of making it easy for the plurality of health professionals to flexibly cooperate according to the condition of the patient. By using the information system 1, for example, information about the patient can be exchanged among health professionals who belong to different medical institutions and who are not acquainted with one another, whereby the health professionals can flexibly cooperate at the time of caring for the patient. The "information about the patient" is, for example, information about medicine to be provided to the patient, information about care for the patient, information about a family circumstance of the patient, or the like. Hereinafter, the information about the patient to be subjected to information exchange will be simply written as "message".

### [Health Professional]

A health professional of a first typical example is a health professional having a specialty in medicine in a specific field and is, for example, a specialized nurse, a certified nurse, a physical therapist, a doctor, a pharmacist, a caregiver, or the like. "Specialty attribute information" indicating a specialty in medicine is associated with the health professional of the first typical example.

The specialty attribute information may be information indicating a specialist qualification regarding medicine. The specialty attribute information may be information indicating a specialty in nursing in a specific field. The specialty attribute information may be information indicating a type of nursing. Examples of the specialty attribute information include: "wound ostomy continence" (WOC) which is a specialism regarding skin or excretion; "stoma care" which is a type of nursing regarding stomata; "palliative care" which is a type of nursing for alleviating physical pain or mental pain from which cancer patients and the like suffer; and "drug therapies" represented by chemotherapies such as anticancer drug therapy.

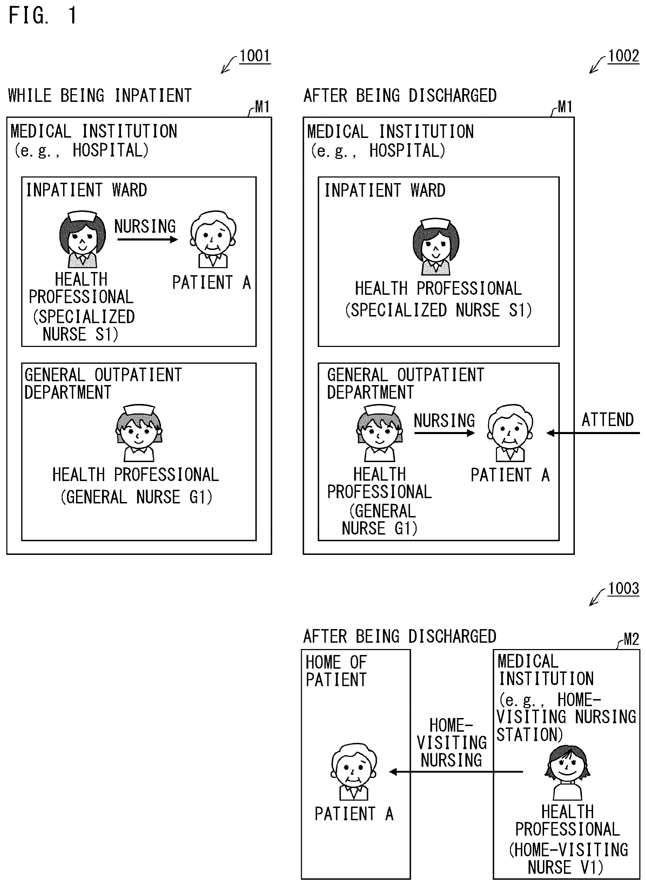

The present embodiment will be described with a specialized nurse being taken as an example of the health professional of the first typical example. The specialized nurse belongs to a medical institution (e.g., hospital) to which a patient has been admitted. The specialized nurse has a specialty in nursing in a specific field and is in charge of nursing the patient who is still an inpatient. In an example indicated by a reference character 1001 in FIG. 1, a specialized nurse S1 belonging to a medical institution M1 is in charge of nursing a patient A admitted to the medical institution M1. The specialized nurse S1 has, for example, a specialty in WOC, and the patient A has undergone, for example, a stoma surgery. After the patient A is discharged, the specialized nurse S1 is not in charge of nursing the patient A anymore.

A health professional of a second typical example is a health professional who does not have a specialty in medicine in a specific field. The present embodiment will be described with (1) a general nurse and (2) a home-visiting nurse being taken as examples of the health professional of the second typical example. The general nurse belongs to the medical institution M1 and is in charge of nursing a patient who attends the general outpatient department of the medical institution M1 after being discharged from the medical institution M1, and the home-visiting nurse belongs to a medical institution M2 (e.g., home-visiting nursing station) different from the medical institution M1, visits the home of the patient discharged from the medical institution M1, and nurses the patient. In an example indicated by a reference character 1002 in FIG. 1, a general nurse G1 belonging to the medical institution M1 is in charge of nursing the patient A who attends the general outpatient department of the medical institution M1 after being discharged. In an example indicated by a reference character 1003 in FIG. 1, a home-visiting nurse V1 belonging to the medical institution M2 is in charge of home-visiting nursing that involves visiting the home of the patient A after the discharge.

The specialized nurse S1 sometimes desires to, for example, transmit WOC-related technical information about the patient A to the general nurse G1 or the home-visiting nurse V1 or ascertain, from the general nurse G1 or the home-visiting nurse V1, a condition of the patient A after the discharge. Likewise, at the time of nursing the patient A, the general nurse G1 or the home-visiting nurse V1 sometimes desires to, for example, consult and take advice from another health professional such as the specialized nurse S1 in relation to WOC and ascertain, from the other health professional, a condition of the patient A obtained while the patient A was an inpatient.

### [Main Advantageous Effect Exhibited through Use of Information System 1]

In a case where a plurality of health professionals are concerned with a target patient in this manner (in particular, a plurality of health professionals belonging to different medical institutions are concerned with the target patient), it has been conventionally difficult to exchange a message among the health professionals unless each of the health professionals knows attribute information (specialties, assigned patients, contact information, and the like) about the other health professionals. However, by using the information system 1, a message regarding a target patient can be easily exchanged among health professionals none of whom knows attribute information about the other health professionals. In particular, the message can be easily exchanged among health professionals who belong to different medical institutions and who are not acquainted with one another. Consequently, the plurality of health professionals can flexibly cooperate according to the condition of the patient.

Specifically, in the information system 1, a group including at least one of "a health professional in charge of a target patient" and "a health professional who has a specialty necessary for providing medicine to the target patient and who belongs to a medical institution associated with the target patient" can be created according to a group creation request from a health professional, and a message can be exchanged in the group. In a case where the latter health professional is included in the group, information involving a high level of expertise can be exchanged in the group. The "medical institution associated with the target patient" is, for example, a medical institution in which medicine requiring a high level of expertise was provided to the target patient, a medical institution in which the target patient underwent a surgery, a medical institution to which the target patient was admitted, or the like.

The group can include a plurality of health professionals in charge of the same patient and/or a plurality of health professionals having a specialty in the same field. Therefore, even in a situation where, for example, a certain health professional in the group is absent or is working so that it is difficult to exchange a message, the message might be able to be exchanged with any of the other health professionals in the group, whereby information exchange is expected to be swiftly performed.

In the information system 1, a group is created according to a group creation request from any of health professionals who are users of the information system 1. Presence of a system manager is not necessary for creating a group. For example, in a case where a home-visiting nurse desires to take advice from a specialized nurse at the time of home-visiting nursing, a group is created according to a request from the home-visiting nurse. Likewise, in a case where a specialized nurse desires to transmit technical information to a general nurse and a home-visiting nurse, a group is created according to a request from the specialized nurse.

### [Example of Group Creation]

An example of group creation will be described with reference to FIG. 2. FIG. 2 shows an example of a group for providing stoma care to the patient A. For example, a group X1 shown in a simplified manner in FIG. 2 can be created as a group including health professionals in charge of the patient A or health professionals each having a specialty in WOC among health professionals belonging to the medical institutions M1 and M2.

The group X1 includes the specialized nurse S1 and a specialized nurse S2. The reason for this is because, although the specialized nurses S1 and S2 are not in charge of the patient A, the specialized nurses S1 and S2 each have the specialty in WOC and belong to the medical institution M1 to which the patient A was admitted. The group X1 preferably includes a specialized nurse who was in charge of the patient A while the patient A was an inpatient in the medical institution M1. The reason for this is because the specialized nurse who was in charge of the patient A is aware of the state of the patient A obtained while the patient A was an inpatient, and thus said specialized nurse easily transmits a message useful for giving care for the patient A. Also, the group X1 preferably includes a plurality of specialized nurses. The reason for this is because, even in a case where one of the specialized nurses cannot immediately answer a question from a general nurse owing to on-going nursing of a patient other than the patient A or the like, another one of the specialized nurses might be able to answer the question. The group X1 further includes the general nurse G1 and the home-visiting nurse V1. The reason for this is because, although the general nurse G1 and the home-visiting nurse V1 do not have the specialty in WOC, the general nurse G1 and the home-visiting nurse V1 are in charge of the patient A.

Meanwhile, a specialized nurse S3 is not included in the group X1. The reason for this is because the specialized nurse S3 is not in charge of the patient A and does not have the specialty in WOC. Likewise, a general nurse G2 and a home-visiting nurse V2 are not included in the group X1. The reason for this is because the general nurse G2 and the home-visiting nurse V2 are not in charge of the patient A and do not have the specialty in WOC.

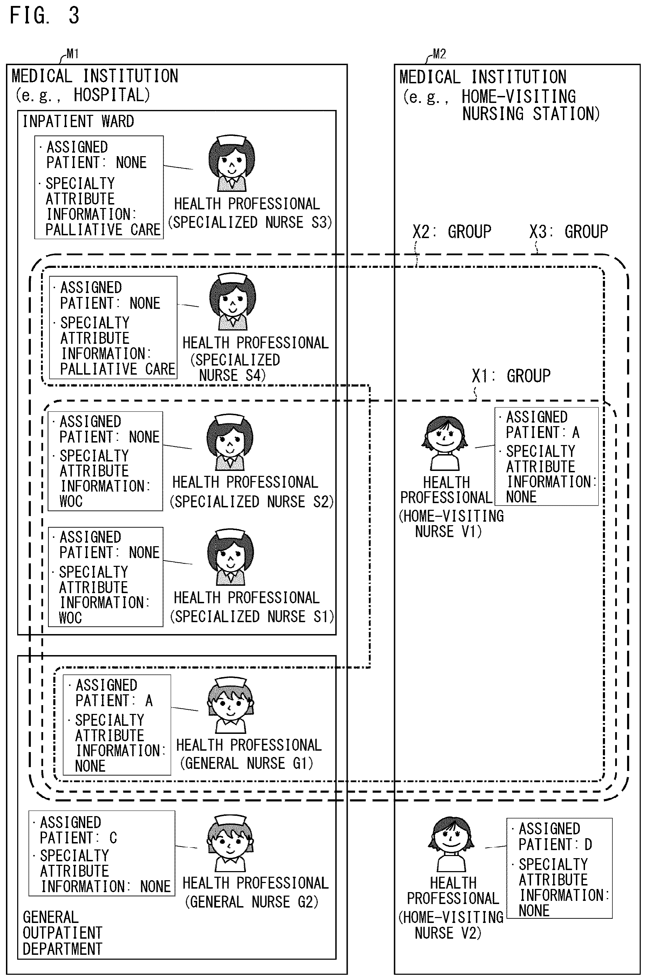

In the information system 1, one or more groups can be created for the same patient. Specifically, in the information system 1, a group can be created for each of pieces of specialty attribute information about medicine to be provided to the patient. Consequently, a message can be exchanged for each of the groups corresponding to the respective specialties. In a case where the pieces of specialty attribute information are information indicating types of nursing, a group can be created for each of the types of nursing to be performed on the patient in the information system 1. For example, in a case where nursing that is palliative care needs to be performed on the patient A in addition to WOC, the above group X1 related to WOC and a group X2 different from the group X1 and related to palliative care can be created for the patient A in the information system 1 as shown in FIG. 3, for example. The group X2 includes a union of: the health professionals in charge of the patient A; and a health professional belonging to the medical institution M1 and having a specialty in palliative care. Specifically, as shown in FIG. 3, the group X2 includes a specialized nurse S4, the general nurse G1, and the home-visiting nurse V1. The specialized nurse S4 is a person who is not in charge of the patient A, but has the specialty in palliative care and belongs to the medical institution M1.

In the information system 1, a group that is for exchanging information about general issues and that is irrespective of the specialties in medicine to be provided to the target patient (hereinafter, referred to as "general group") may be further created. The general issues refer to, for example, issues related to a family circumstance, a financial circumstance, and the like of the target patient. A general group of a first typical example is a group including all the health professionals in charge of the target patient. A general group of a second typical example is a group including all the health professionals included in at least one of the plurality of groups having been created for the target patient. A group X3 shown in FIG. 3 is a general group of the second typical example for the patient A. The group X3 includes the specialized nurse S1, the specialized nurse S2, the specialized nurse S4, the general nurse G1, and the home-visiting nurse V1.

The general group may be created at, for example, a timing at which a group for the target patient is created. For example, the group X3 may be created each time either of the group X1 and the group X2 is created. Alternatively, the general group may be created according to, for example, a creation request made by a medical worker.

### [Thread]

In the information system 1, a "thread" which is a set of posted messages is created for each of the groups. In the present embodiment, different threads are created for the respective groups. That is, one group corresponds to one thread.

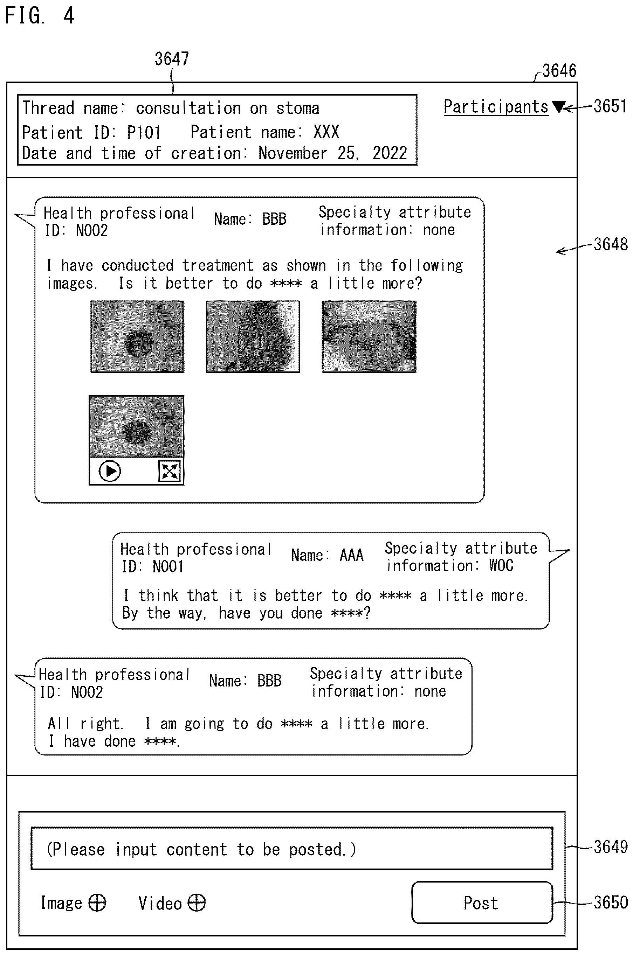

Health professionals included in a same group can perform information exchange by posting messages in the same corresponding thread. The information exchange can be performed in a conversational style by using a chat function, for example. An example of a thread screen displayed on a terminal 3 being used by a health professional is shown in FIG. 4. In a thread screen 3646 shown in FIG. 4, messages regarding a patient having a patient ID of "P101" are exchanged between a health professional having a health professional ID of "N001" and a health professional having a health professional ID of "N002" in a field 3648. The thread screen 3646 will be described later in detail.

### [Configuration Example of Information System 1]

As shown in FIG. 2, the information system 1 has a configuration including a server 2 and a plurality of terminals 3. The server 2 is a computer possessed by a business operator operating the information system 1 and is an information processing device playing a pivotal role in the information system 1. The server 2 may be implemented by one computer or may be implemented by a plurality of computers among which functions are distributed. The terminals 3 are computers being used by respective health professionals utilizing the information system 1 and are each, for example, a smartphone, a tablet terminal, or a personal computer. The server 2 and each of the terminals 3 are communicably connected via a communication network 4 such as the Internet. Transmission and reception of messages among the terminals 3 are performed via the server 2 and the communication network 4.

### [Configuration Example of Server 2]

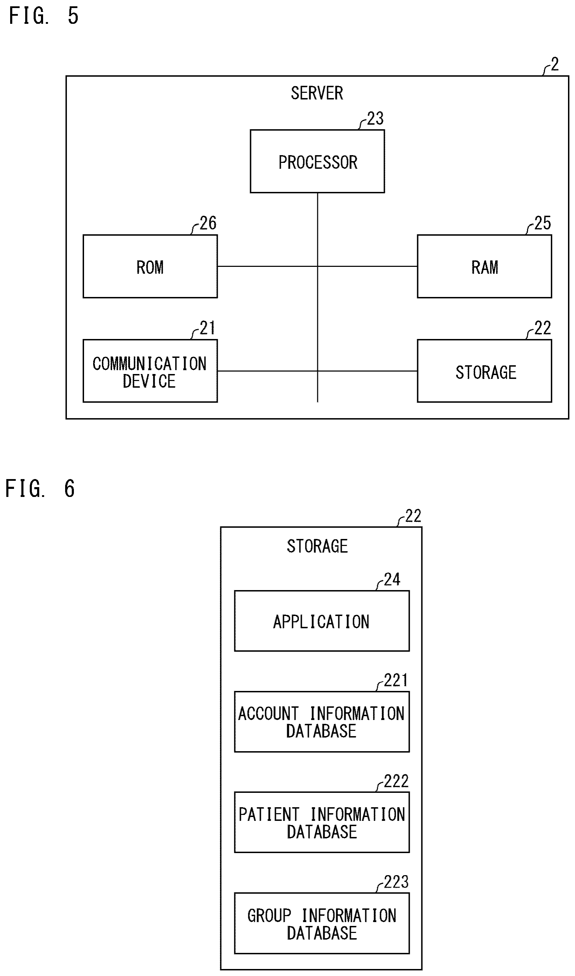

As shown in FIG. 5, the server 2 includes, for example, a communication device 21, a storage 22, a processor 23, a random access memory (RAM) 25, and a read only memory (ROM) 26. The communication device 21 is an interface through which communication with the terminals 3 is performed according to control by the processor 23. The storage 22 is a memory for storing therein various data and various programs to be used by the server 2 and is, for example, a semiconductor drive such as a solid-state drive or a magnetic disk such as a hard disk. As shown in FIG. 6, the storage 22 stores therein, for example, an account information database 221, a patient information database 222, and a group information database 223.

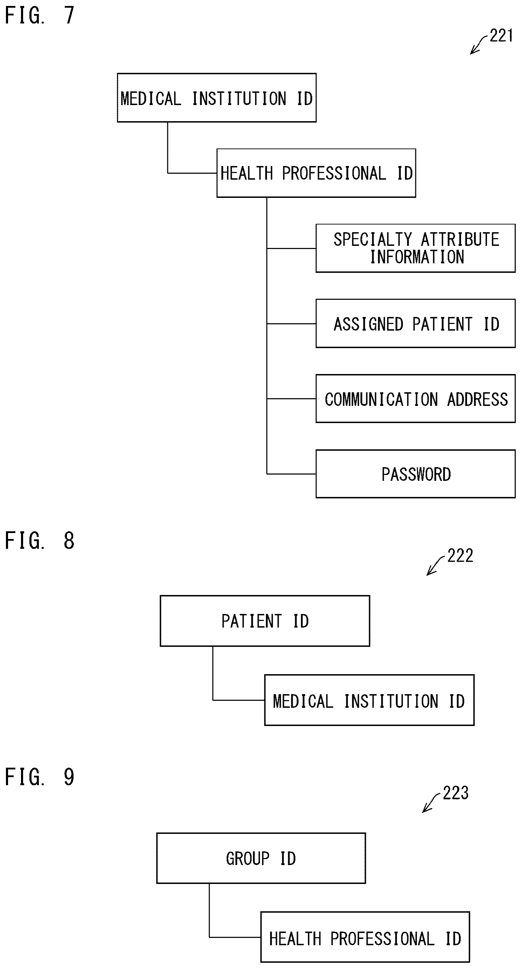

The account information database 221 is a database for storing therein account information about each of the health professionals utilizing the information system 1 and is, for example, a relational database (RDB). FIG. 7 shows an example of a data structure in a case where the account information database 221 is an RDB. As shown in FIG. 7, the account information includes, for example, a medical institution ID, a health professional ID, specialty attribute information, an assigned patient ID, a communication address, and a password.

The medical institution ID is identification information for a medical institution to which the health professional belongs. One or more health professional IDs are associated with the medical institution ID. Another piece of attribute information about the medical institution (e.g., the name of the medical institution or the like) may be associated with the medical institution ID.

Each of the health professional IDs is identification information for the corresponding health professional belonging to the medical institution identified by the medical institution ID. As shown in FIG. 7, for example, specialty attribute information, an assigned patient ID, a communication address, and a password are associated with the health professional ID. Another piece of attribute information about the health professional (e.g., the name of the health professional or the like) may be associated with the health professional ID.

The specialty attribute information is identification information for identifying a specialty of the health professional identified by the health professional ID. In a case where the health professional has a plurality of specialties, a plurality of pieces of specialty attribute information are associated with the health professional ID. In a case where the health professional does not have a specialty in medicine in a specific field, the value of the specialty attribute information associated with the health professional ID is set to, for example, a null value (NULL).

The assigned patient ID is identification information for a patient of whom the health professional identified by the health professional ID is in charge. In a case where the health professional is in charge of a plurality of patients, a plurality of assigned patient IDs are associated with the health professional ID. In a case where the health professional is not in charge of any patient, the value of the assigned patient ID associated with the health professional ID is set to, for example, a null value (NULL).

The communication address is a communication address of the terminal 3 being used by the health professional identified by the health professional ID and is, for example, an IP address. The password is a password with which the health professional identified by the health professional ID logs in to the information system 1.

The patient information database 222 is a database for storing therein patient information and is, for example, an RDB. FIG. 8 shows an example of a data structure in a case where the patient information database 222 is an RDB. As shown in FIG. 8, the patient information includes, for example, a patient ID and a medical institution ID.

The patient ID is identification information for a patient and is based on the same coding scheme as that of the patient ID managed in the account information database 221. The medical institution ID is associated with the patient ID. The medical institution ID is identification information for a medical institution in charge of providing medicine to the patient identified by the patient ID. The medical institution indicated by the medical institution ID is, for example, a medical institution in which medicine requiring a high level of expertise was provided to the patient indicated by the patient ID, a medical institution in which the patient indicated by the patient ID underwent a surgery, a medical institution to which the patient indicated by the patient ID was admitted, or the like. The medical institution ID managed in the patient information database 222 is based on the same coding scheme as that of the medical institution ID managed in the account information database 221. Another piece of attribute information about the patient (e.g., the name of the patient or the like) may be associated with the patient ID.

The group information database 223 is a database for storing therein information about a group of health professionals among whom a message is exchanged and is, for example, an RDB. FIG. 9 shows an example of a data structure in a case where the group information database 223 is an RDB. As shown in FIG. 9, the information about the group includes, for example, a group ID and a health professional ID.

The group ID is identification information for the group. A plurality of health professional IDs are associated with the group ID. Each of the health professional IDs is identification information for the corresponding health professional included in the group identified by the group ID and is based on the same coding scheme as that of the health professional ID managed in the account information database 221. Another piece of attribute information about the group (e.g., the name of the group or the like) may be associated with the group ID.

The processor 23 is a controller that comprehensively controls the functions of the server 2. The processor 23 is, for example, a central processing unit (CPU). The processor 23 may be implemented by, for example, a logic circuit. The processor 23 reads out an application 24 from the storage 22, loads the application 24 to the RAM 25, and executes the application 24. The application 24 is stored in, for example, the storage 22 as a program for causing a computer to function as a controller that executes various processes. The application 24 is, for example, an application dedicated to the information system 1. The application 24 is distributed or downloaded to the server 2 from an application-providing server, is installed in the server 2, and is stored in the storage 22, for example.

### [Configuration Example of Application 24]

FIG. 10 shows a configuration example of the application 24. The application 24 includes, for example, an account management unit 241, a patient management unit 242, a group management unit 243, and an information exchange unit 244.

The processor 23 follows an instruction from the account management unit 241 to register account information according to a request from any of the terminals 3. Specifically, first, the processor 23 follows an instruction from the account management unit 241 to receive an account registration request. The account registration request is transmitted, according to an input operation performed by any of the health professionals, from the corresponding terminal 3 and includes, for example, account information such as a health professional ID, a password, a medical institution ID, specialty attribute information, and a patient ID as described later. The processor 23 follows an instruction from the account management unit 241 to store, in the account information database 221, the account information included in the account registration request. A communication address in the account information may be transmitted from the terminal 3 regardless of the account registration request each time any of the health professionals logs in to the information system 1 by using the corresponding terminal 3, for example. In this case, the processor 23 may follow an instruction from the account management unit 241 to update the account information database 221 each time a communication address is received from any of the terminals 3.

The processor 23 follows an instruction from the patient management unit 242 to register patient information according to a request from any of the terminals 3. Specifically, first, the processor 23 follows an instruction from the patient management unit 242 to receive a patient registration request transmitted from any of the terminals 3. The patient registration request is transmitted, according to an input operation performed by any of the health professionals who desires to register patient information, from the corresponding terminal 3 and includes, for example, a patient ID and a medical institution ID as described later. The processor 23 follows an instruction from the patient management unit 242 to store, in the patient information database 222, the patient information included in the patient registration request.

The processor 23 follows an instruction from the group management unit 243 to create a group of health professionals, among whom information is exchanged, according to a request from any of the terminals 3. A group creation process will be described below.

First, the processor 23 follows an instruction from the group management unit 243 to receive a group creation request (hereinafter, simply referred to as "creation request") from any of the terminals 3. The creation request is transmitted, according to an input operation performed by any of the health professionals, from the corresponding terminal 3 and is a request based on, for example, a patient ID and specialty attribute information as described later. The processor 23 follows an instruction from the group management unit 243 to: receive the creation request; and, upon the reception, search the patient information database 222 for a medical institution ID associated with the target patient ID. Subsequently, the processor 23 follows an instruction from the group management unit 243 to search the account information database 221 for a set of health professional IDs. The searching pattern used at this time is, for example, a searching pattern Q1 or a searching pattern Q2 described below.

(Searching pattern Q1) Health professionals of the first typical example who have the specialty specified in the creation request regardless of whether or not the health professionals are in charge of the patient specified in the creation request, and health professionals of the second typical example who are in charge of the specified patient, are searched for. Specifically, a set of health professional IDs which is a union of search targets Q11 and Q12 described below is obtained from the account information database 221. [Search target Q11] Health professional IDs with which the specialty attribute information included in the received creation request is associated among health professional IDs associated with the medical institution ID obtained from the patient information database 222. [Search target Q12] Health professional IDs with which the patient ID included in the received creation request is associated.

(Searching pattern Q2) Health professionals of the first typical example who have the specialty specified in the creation request and who are in charge of the patient specified in the creation request, and health professionals of the second typical example who are in charge of the specified patient, are searched for. Specifically, a set of health professional IDs which is a union of search targets Q21 and Q22 described below is obtained from the account information database 221. [Search target Q21] Health professional IDs with which the specialty attribute information included in the received creation request and the patient ID included in the received creation request are associated among the health professional IDs associated with the medical institution ID obtained from the patient information database 222. [Search target Q22] Health professional IDs with which the specialty attribute information included in the received creation request is not associated and the patient ID included in the received creation request is associated among the health professional IDs associated with the medical institution ID obtained from the patient information database 222.

Meanwhile, in a case where the specialty attribute information included in the creation request has a NULL value, the group management unit 243 causes searching of the account information database 221 for a set of health professional IDs by using a searching pattern Q3 described below.

(Searching pattern Q3) All the health professionals in charge of the specified patient are searched for. Specifically, a set of health professional IDs with which the patient ID included in the received creation request is associated is obtained from the account information database 221.

Subsequently, the processor 23 follows an instruction from the group management unit 243 to: newly create a group in which, with the set of health professional IDs obtained from the account information database 221, a group ID for identifying the set of health professional IDs as one group has been associated; and store the newly created group in the group information database 223.

A group composed of the set of health professional IDs searched for by using the searching pattern Q3 is a group that is related to the target patient and that is irrespective of the specific specialty. This group corresponds to the above "general group". Therefore, at a timing of creating a group composed of the set of health professional IDs obtained by using the searching pattern Q1 or Q2, the processor 23 may follow an instruction from the group management unit 243 to create a general group composed of the set of health professional IDs obtained by using the searching pattern Q3.

Next, the processor 23 follows an instruction from the group management unit 243 to search the account information database 221 so as to obtain communication addresses associated with the respective health professional IDs included in the newly created group. Subsequently, the processor 23 follows an instruction from the group management unit 243 to notify each of the terminals 3 having the obtained respective communication addresses that the group has been created (hereinafter, simply referred to as "creation notification"). The creation notification includes: the group ID of the created group; and the patient ID and the specialty attribute information included in the received creation request.

The processor 23 follows an instruction from the information exchange unit 244 to: receive message information about a message to be subjected to information exchange from any of the terminals 3; and, upon the reception, execute a server-side information exchange process. The message information includes, for example, a message ID, a thread ID, a health professional ID, and a data body as described later.

The server-side information exchange process will be described below. First, the processor 23 follows an instruction from the information exchange unit 244 to search the group information database 223 for health professional IDs associated with a group ID corresponding to the thread ID included in the received message information. Subsequently, the processor 23 follows an instruction from the information exchange unit 244 to search the account information database 221 so as to obtain the communication addresses associated with the respective health professional IDs obtained from the group information database 223. Subsequently, the processor 23 follows an instruction from the information exchange unit 244 to transmit the received message information to each of the terminals 3 having the obtained respective communication addresses (excluding the terminal 3 which is the transmission source of the message information). The timing of transmitting the message information may be a timing immediately subsequent to said reception or a timing at which the terminals 3 as transmission destinations access the information system 1.

### [Configuration Example of Terminal 3]

As shown in FIG. 11, each of the terminals 3 includes, for example, a communication device 31, an input device 32, an output device 33, a storage 34, a processor 35, a RAM 37, and a ROM 38. The communication device 31 is an interface through which communication with the server 2 is performed according to control by the processor 35. The input device 32 accepts input of various data to the terminal 3. The input device 32 is, for example, a touch panel, a camera, or a microphone. The output device 33 outputs various data processed by the terminal 3. The output device 33 is, for example, a touch panel, a speaker, or a printer.

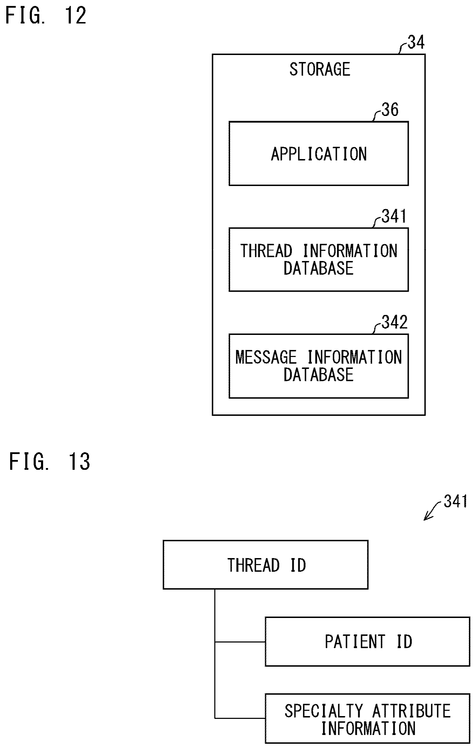

The storage 34 is a memory for storing therein various data and various programs to be used by the terminal 3 and is, for example, a semiconductor drive such as a solid-state drive or a magnetic disk such as a hard disk. As shown in FIG. 12, the storage 34 stores therein, for example, a thread information database 341 and a message information database 342.

The thread information database 341 is a database for storing therein thread information and is, for example, an RDB. FIG. 13 shows an example of a data structure in a case where the thread information database 341 is an RDB. As shown in FIG. 13, the thread information includes, for example, a thread ID, a patient ID, and specialty attribute information.

The thread ID is identification information for a thread. In the present embodiment, one thread ID corresponds to one group ID managed in the group information database 223. The thread ID and the group ID may be based on a same coding scheme or mutually convertible coding schemes.

As shown in FIG. 13, the patient ID and the specialty attribute information are associated with the thread ID. Another piece of attribute information about the thread (e.g., the name of the thread or the like) may be associated with the thread ID.

The patient ID is identification information for a patient regarding whom information exchange is to be performed in the thread identified by the thread ID. The patient ID is based on the same coding scheme as that of the patient ID managed in the patient information database 222. The specialty attribute information is specialty attribute information regarding which information exchange is to be performed in the thread identified by the thread ID and is based on the same coding scheme as that of the specialty attribute information managed in the account information database 221.

The message information database 342 is a database for storing therein message information and is, for example, an RDB. FIG. 14 shows an example of a data structure in a case where the message information database 342 is an RDB. As shown in FIG. 14, the message information includes, for example, a message ID, a thread ID, a health professional ID, and a data body.

The message ID is identification information for the message information. As shown in FIG. 14, the thread ID, the health professional ID, and the data body are associated with the message ID. Another piece of attribute information about the message (e.g., the date and time of transmission of the message or the like) may be associated with the message ID.

The thread ID is identification information for the thread in which the message identified by the message ID is subjected to information exchange. The thread ID is based on the same coding scheme as that of the thread ID managed in the thread information database 341. The health professional ID is identification information for the health professional who has transmitted the message identified by the message ID. The health professional ID is based on the same coding scheme as that of the health professional ID managed in the account information database 221. The data body is data itself of the message identified by the message ID. The data format of the data body is not limited and is, for example, at least one of text data, still image data, moving image data, and voice data.

The processor 35 is a controller that comprehensively controls the functions of the terminal 3. The processor 35 is, for example, a CPU. The processor 35 may be implemented by, for example, a logic circuit. The processor 35 reads out an application 36 from the storage 34, loads the application 36 to the RAM 37, and executes the application 36. The application 36 is stored in, for example, the storage 34 as a program for causing a computer to function as a controller that executes various processes. The application 36 is, for example, an application dedicated to the information system 1. The application 36 is distributed or downloaded to the terminal 3 from an application-providing server, is installed in the terminal 3, and is stored in the storage 34, for example. The application 36 may be, for example, provided as a web application by the application-providing server and operated on a web browser installed in the terminal 3.

### [Configuration Example of Application 36]

FIG. 15 shows a configuration example of the application 36. The application 36 includes, for example, an account registration unit 361, a patient registration unit 362, a thread management unit 363, and an information exchange unit 364.

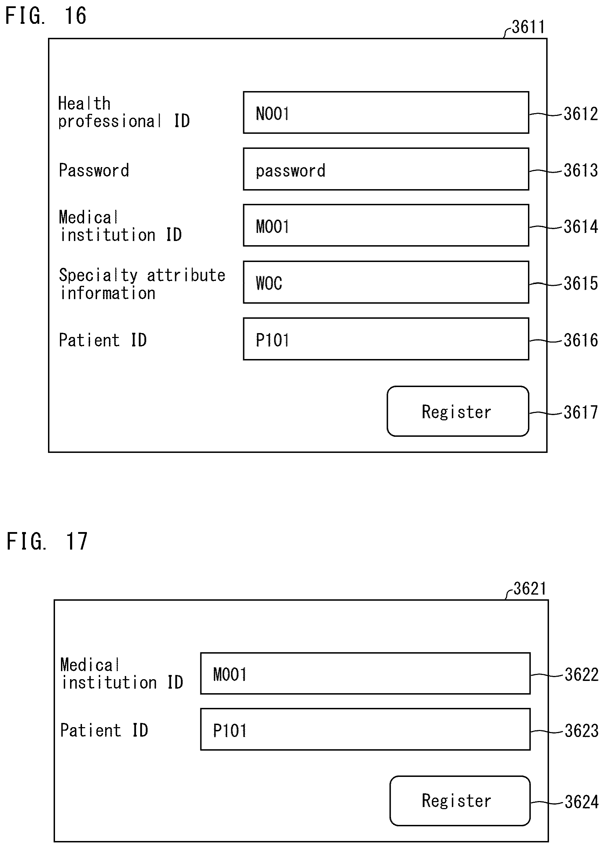

The processor 35 follows an instruction from the account registration unit 361 to: create an account registration screen 3611 according to an input operation performed by the health professional; and cause the output device 33 to display the account registration screen 3611. The account registration screen 3611 is a screen for accepting new registration or updating registered account information. FIG. 16 shows an example of the account registration screen 3611. As shown in FIG. 16, the account registration screen 3611 includes, for example, fields 3612 to 3616 and a button 3617.

In the field 3612, input of a health professional ID of the health professional to be registered is accepted. In the field 3612, input of the health professional ID may be accepted from the health professional using the terminal 3, or the health professional ID may be automatically set. An example of the automatic setting may be such that the processor 35 follows an instruction from the account registration unit 361 to: transmit a health professional ID-providing request to the server 2 at the time of displaying the account registration screen 3611; and set, in the field 3612, a health professional ID obtained from the server 2 in response to the transmission.

In the field 3613, input of a password with which the health professional to be registered logs in to the information system 1 is accepted.

In the field 3614, input of a medical institution ID of a medical institution to which the health professional to be registered belongs is accepted. The field 3614 is preferably of a selection style. For example, any of medical institution names displayed in a drop-down list prepared in advance or the like is preferably selected to automatically set, in the field 3614, a medical institution ID corresponding to the selected medical institution name.

In the field 3615, input of a piece of specialty attribute information about the health professional to be registered is accepted. The number of the fields 3615 can be increased or decreased according to the number of pieces of the specialty attribute information. The field 3615 is preferably of a selection style. Any of pieces of specialty attribute information displayed in a drop-down list prepared in advance or the like is preferably selected to automatically set, in the field 3615, the piece of specialty attribute information. The field 3615 does not necessarily have to be filled. Account information about a health professional who does not have any specialty in medicine can be registered without filling the field 3615.

In the field 3616, input of a patient ID of a patient of whom the health professional to be registered is in charge is accepted. The number of the fields 3616 can be increased or decreased according to the number of patients of whom the health professional to be registered is in charge. The field 3616 does not necessarily have to be filled. Account information about a health professional who is not in charge of any patient can be registered without filling the field 3616.

When accepting an operation through the button 3617, the processor 35 follows an instruction from the account registration unit 361 to transmit an account registration request including the pieces of account information inputted in the fields 3612 to 3616 to the server 2.

The processor 35 follows an instruction from the patient registration unit 362 to: create a patient registration screen 3621 according to an input operation performed by the health professional; and cause the output device 33 to display the patient registration screen 3621. The patient registration screen 3621 is a screen for accepting new registration or updating registered patient information. FIG. 17 shows an example of the patient registration screen 3621. As shown in FIG. 17, the patient registration screen 3621 includes, for example, fields 3622 and 3623 and a button 3624.

In the field 3622, input of a medical institution ID of a medical institution associated with the patient to be registered is accepted. The field 3622 is preferably of a selection style and more preferably allows a medical institution ID to be automatically set. An example of the automatic setting may be such that the processor 35 follows an instruction from the patient registration unit 362 to: transmit a search request for a medical institution ID to the server 2 at the time of displaying the patient registration screen 3621; and set, in the field 3622, a medical institution ID obtained from the server 2 in response to the transmission. In this case, the server 2 may: search the account information database 221 so as to obtain a medical institution ID associated with the health professional ID of the health professional who has logged in to the information system 1 from the terminal 3 with which the search request has been made; and transmit the medical institution ID to the terminal 3 with which the search request has been made.

In the field 3623, input of a patient ID of the patient to be registered is accepted. The patient ID to be inputted in the field 3623 may be a patient ID itself managed in the patient information database 222 or may be a number indicated in a patient registration ticket or an ID of a medical record issued by a medical institution to which the patient to be registered was admitted or that said patient attended. In the latter case, information obtained by combining the medical institution ID inputted in the field 3622 and the patient ID inputted in the field 3623 may be treated as a patient ID to be managed in the patient information database 222.

When accepting an operation through the button 3624, the processor 35 follows an instruction from the patient registration unit 362 to transmit a patient registration request including the pieces of patient information inputted in the fields 3622 and 3623 to the server 2.

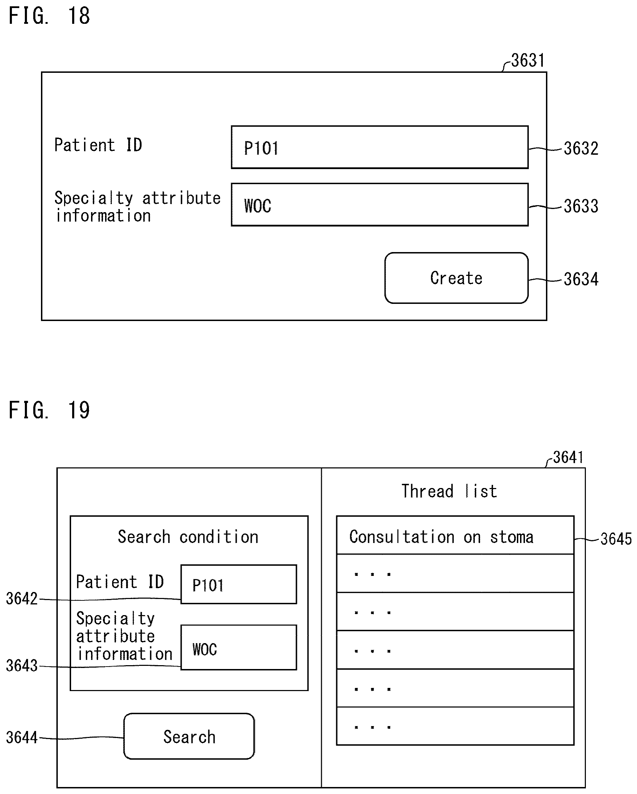

The processor 35 follows an instruction from the thread management unit 363 to: create a creation request screen 3631 for requesting creation of a group according to an input operation performed by the health professional; and cause the output device 33 to display the creation request screen 3631. The creation request screen 3631 is a screen for accepting a creation request. FIG. 18 shows an example of the creation request screen 3631. As shown in FIG. 18, the creation request screen 3631 includes, for example, fields 3632 and 3633 and a button 3634.

In the field 3632, input of a patient ID of the patient regarding whom information exchange is to be performed is accepted. In the field 3633, input of specialty attribute information about medicine to be provided to the patient indicated by the patient ID inputted in the field 3632 is accepted. The field 3633 does not necessarily have to be filled.

It is assumed that the health professional who makes the creation request can obtain the patient ID and the specialty attribute information in advance. For example, a home-visiting nurse in charge of home-visiting nursing of a patient obtains a patient ID and specialty attribute information in advance by means of an instruction document, e-mail, or the like sent from a medical institution in which the patient underwent therapy. The field 3622 may be of a selection style. For example, assigned patient IDs associated with the health professional ID of the health professional who makes the creation request may be obtained from the account information database 221 and may be displayed in a drop-down list.

When accepting an operation through the button 3634, the processor 35 follows an instruction from the thread management unit 363 to transmit a creation request including the pieces of information inputted in the fields 3632 and 3633 to the server 2. In a case where nothing is inputted in the field 3633, NULL is set as the value of the specialty attribute information.

In addition, the processor 35 follows an instruction from the thread management unit 363 to: receive a creation notification from the server 2; and, upon the reception, create and manage a thread. Specifically, the processor 35 follows an instruction from the thread management unit 363 to: create thread information by associating the patient ID and the specialty attribute information included in the creation notification with a thread ID corresponding to a group ID included in the creation notification; and store the created thread information in the thread information database 341.

The processor 35 follows an instruction from the information exchange unit 364 to execute a terminal-side information exchange process. The terminal-side information exchange process includes a message-transmission-side process and a message-reception-side process. Firstly, the message-transmission-side process will be described.

The processor 35 follows an instruction from the information exchange unit 364 to: create a thread selection screen 3641 according to an input operation performed by the health professional; and cause the output device 33 to display the thread selection screen 3641. The thread selection screen 3641 is a screen that allows the health professional to select a desired thread. FIG. 19 shows an example of the thread selection screen 3641. As shown in FIG. 19, the thread selection screen 3641 includes, for example, fields 3642 and 3643, a button 3644, and a list 3645.

In the fields 3642 and 3643, input of conditions for searching for a desired thread is accepted. In the field 3642, input of a patient ID is accepted. In the field 3643, input of a piece of specialty attribute information is accepted. The field 3643 is preferably of a selection style. Any of pieces of specialty attribute information displayed in a drop-down list prepared in advance or the like is preferably selected to automatically set, in the field 3643, the piece of specialty attribute information.

When accepting an operation through the button 3644, the processor 35 follows an instruction from the information exchange unit 364 to: search the thread information database 341 so as to obtain threads having thread IDs associated with the patient ID and the specialty attribute information inputted in the fields 3642 and 3643; and cause the obtained threads to be listed in the list 3645. In an example shown in FIG. 19, threads having thread IDs associated with a patient ID "P101" and specialty attribute information "WOC" are listed in the list 3645.

When accepting a thread selection operation in the list 3645, the processor 35 follows an instruction from the information exchange unit 364 to: search the message information database 342; create a thread screen 3646 corresponding to the selected thread; and cause the output device 33 to display the thread screen 3646. FIG. 4 shows an example of the thread screen 3646. As shown in FIG. 4, the thread screen 3646 includes, for example, fields 3647 to 3649 and buttons 3650 and 3651.

In the field 3647, pieces of information about the thread are displayed. In an example shown in FIG. 4, the name of the thread, the patient ID, the name of the patient, and the date and time of the creation are displayed as pieces of information about the thread. In the field 3648, messages subjected to information exchange are displayed in time series. Each of the messages is a data body stored in the message information database 342 and may be text, a still image, or a moving image. In the example shown in FIG. 4, still images each showing a lesion of the patient and a moving image showing the lesion of the patient are displayed as the data body in addition to text indicating a consultation issue. In the field 3649, posting of a new message is accepted. The message to be accepted may be text, a still image, or a moving image.

When accepting an operation through the button 3650, the processor 35 follows an instruction from the information exchange unit 364 to transmit the message information to the server 2. The message information includes: the data body inputted in the field 3649; the thread ID of the thread; the health professional ID of the health professional who has posted the message; and a message ID for identifying the message information. In addition, the processor 35 follows an instruction from the information exchange unit 364 to manage the message information transmitted to the server 2. Specifically, the processor 35 follows an instruction from the information exchange unit 364 to store the message information, which has been transmitted to the server 2, in the message information database 342.

The button 3651 is a toggle button for accepting an operation for switching between displaying and hiding of pieces of attribute information (the health professional IDs, the names, and the like) about the health professionals among whom information exchange can be performed in the thread. The pieces of attribute information are obtained from, for example, the server 2. Specifically, the processor 35 follows an instruction from the information exchange unit 364 to transmit a health professional obtainment request including the thread ID to the server 2. The server 2 searches the group information database 223 so as to obtain health professional IDs associated with a group ID corresponding to the thread ID included in the health professional obtainment request. Subsequently, the server 2 searches the account information database 221 so as to obtain pieces of attribute information, about the health professionals, with which the health professional IDs obtained from the group information database 223 are associated. Then, the server 2 transmits the obtained pieces of attribute information to the terminal 3 from which the health professional obtainment request has been transmitted.

Next, the message-reception-side process will be described. The processor 35 follows an instruction from the information exchange unit 364 to: receive the message information from the server 2; and, upon the reception, manage the received message information. Specifically, the information exchange unit 364 stores, in the message information database 342, the message ID, the thread ID, the health professional ID, and the data body which are included in the message information, for example.

The processor 35 follows an instruction from the information exchange unit 364 to: receive the message information while the thread screen 3646 is displayed; and, upon the reception, update displayed content of the field 3648 according to the received message information.

Meanwhile, the processor 35 may follow an instruction from the information exchange unit 364 to: receive the message information while the thread screen 3646 is not displayed; and, upon the reception, cause the output device 33 to display notification information indicating that the message information has been received. The notification information is given in the form of, for example, a notification dot, a pop-up notification, a banner notification, or the like. The notification information makes it easy for the health professional to notice that a message has been received. The processor 35 may follow an instruction from the information exchange unit 364 to cause the output device 33 to display the thread screen 3646 according to an operation performed on the displayed notification information by the health professional.

### [Example of Flow of Process for Creating Group and Thread]

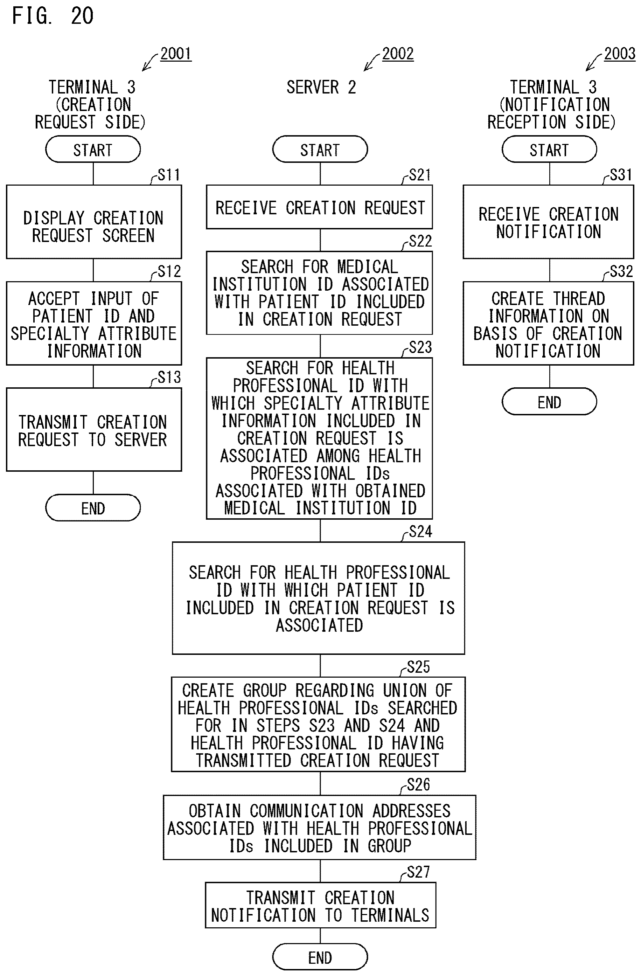

FIG. 20 shows flowcharts indicating an example of the flow of a process for creating a group and a thread, the process being a part of an information exchange method according to the present embodiment. Firstly, a process that is performed by any of the terminals 3 from which a creation request is to be transmitted will be described with reference to a flowchart indicated by a reference character 2001. Each of steps shown in the flowchart indicated by the reference character 2001 is a step to be executed by the processor 35 of the terminal 3. First, the processor 35 follows an instruction from the thread management unit 363 to cause the output device 33 to display the creation request screen 3631 according to an input operation performed by the corresponding health professional (S11). Subsequently, the processor 35 follows an instruction from the thread management unit 363 to: accept input of a patient ID and specialty attribute information in the creation request screen 3631 (S12); and transmit a creation request including the inputted pieces of information to the server 2 (S13).

Next, a process that is performed by the server 2 will be described with reference to a flowchart indicated by a reference character 2002. Each of steps shown in the flowchart indicated by the reference character 2002 is a step to be executed by the processor 23 of the server 2. The processor 23 follows an instruction from the group management unit 243 to: receive the creation request transmitted in the above step S13 (S21); and, upon the reception, search the patient information database 222 for a medical institution ID associated with the patient ID included in the received creation request (S22). Subsequently, the processor 23 follows an instruction from the group management unit 243 to search for health professionals satisfying the received creation request in steps S22 to S24. Hereinafter, description will be given regarding a case where the processor 23 follows an instruction from the group management unit 243 to search the account information database 221 by using the above searching pattern Q1. In this case, the processor 23 follows an instruction from the group management unit 243 to search the account information database 221 for a health professional ID with which the specialty attribute information included in the received creation request is associated among health professional IDs associated with the medical institution ID obtained from the patient information database 222 (S23). In addition, the processor 23 follows an instruction from the group management unit 243 to search the account information database 221 for a health professional ID with which the patient ID included in the received creation request is associated (S24). Subsequently, the processor 23 follows an instruction from the group management unit 243 to create a group in which a newly created group ID has been associated with a union of the health professional IDs searched for in steps S23 and S24 and the health professional ID of the health professional having transmitted the creation request and store the group in the group information database 223 (S25). Subsequently, the processor 23 follows an instruction from the group management unit 243 to search the account information database 221 so as to obtain communication addresses associated with the respective health professional IDs included in the created group (S26). Subsequently, the processor 23 follows an instruction from the group management unit 243 to transmit a creation notification to each of the terminals 3 having the obtained respective communication addresses (S27).

Next, a process that is performed by each of the terminals 3 having received the creation notification will be described with reference to a flowchart indicated by a reference character 2003. Each of steps shown in the flowchart indicated by the reference character 2003 is a step to be executed by the processor 35 of the terminal 3. The processor 35 follows an instruction from the thread management unit 363 to: receive the creation notification transmitted in the above step S27 (S31); and, upon the reception, create thread information on the basis of the creation notification (S32). Specifically, the processor 35 follows an instruction from the thread management unit 363 to: create thread information by associating the patient ID and the specialty attribute information included in the creation notification with a thread ID corresponding to the group ID included in the creation notification; and store the created thread information in the thread information database 341.

### [Example of Flow of Information Exchange Process]

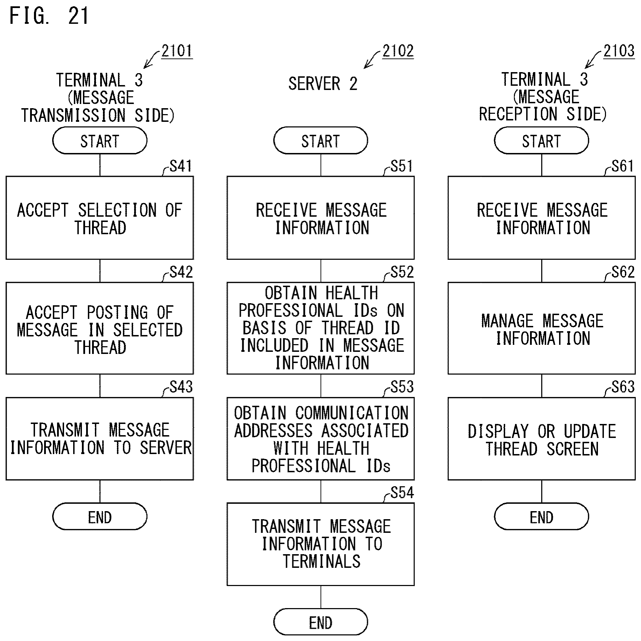

FIG. 21 shows flowcharts indicating an example of the flow of an information exchange process which is a part of the information exchange method according to the present embodiment. Firstly, a process that is performed by any of the terminals 3 from which a message is to be transmitted will be described with reference to a flowchart indicated by a reference character 2101. Each of steps shown in the flowchart indicated by the reference character 2101 is a step to be executed by the processor 35 of the terminal 3. First, the processor 35 follows an instruction from the information exchange unit 364 to: cause the output device 33 to display the thread selection screen 3641 according to an input operation performed by the corresponding health professional; and accept selection of a thread (S41). Subsequently, the processor 35 follows an instruction from the information exchange unit 364 to cause the output device 33 to display a thread screen 3646 corresponding to the selected thread and accept posting of a message (S42). Subsequently, the processor 35 follows an instruction from the information exchange unit 364 to store, in the message information database 342, message information including the accepted message and transmit the message information to the server 2 (S43).

Next, a process that is performed by the server 2 will be described with reference to a flowchart indicated by a reference character 2102. Each of steps shown in the flowchart indicated by the reference character 2102 is a step to be executed by the processor 23 of the server 2. The processor 23 follows an instruction from the information exchange unit 244 to: receive the message information transmitted in the above step S43 (S51); and, upon the reception, search the group information database 223 so as to obtain health professional IDs associated with a group ID corresponding to a thread ID included in the received message information (S52). Subsequently, the processor 23 follows an instruction from the information exchange unit 244 to search the account information database 221 so as to obtain communication addresses associated with the respective health professional IDs obtained from the group information database 223 (S53). Subsequently, the processor 23 follows an instruction from the information exchange unit 244 to transmit the received message information to each of the terminals 3 having the obtained respective communication addresses (excluding the terminal 3 which is the transmission source of the message information) (S54).

Next, a process that is performed by each of the terminals 3 having received the message information will be described with reference to a flowchart indicated by a reference character 2103. Each of steps shown in the flowchart indicated by the reference character 2103 is a step to be executed by the processor 35 of the terminal 3. The processor 35 follows an instruction from the information exchange unit 364 to: receive the message information transmitted from the server 2 in the above step S54 (S61); and, upon the reception, manage the received message information (S62). Specifically, the processor 35 follows an instruction from the information exchange unit 364 to store, in the message information database 342, the message ID, the thread ID, the health professional ID, and the data body which are included in the message information, for example. Then, the processor 35 follows an instruction from the information exchange unit 364 to, according to the received message information, cause the output device 33 to display a thread screen or update the thread screen displayed on the output device 33, for example (S63). The processor 35 may follow an instruction from the information exchange unit 364 to cause the output device 33 to display notification information indicating that the message information has been received.

### [Modification 1: Video Call]

The information system 1 may have a function of allowing execution of a video call among the health professionals in the group. Execution of a video call among the health professionals makes it possible to perform information exchange while visually recognizing the condition of the patient. The video call function may include, in addition to a function of transmitting and receiving a moving image, a function of causing a character or an image to be displayed so as to be superimposed on the moving image by using a paint tool.

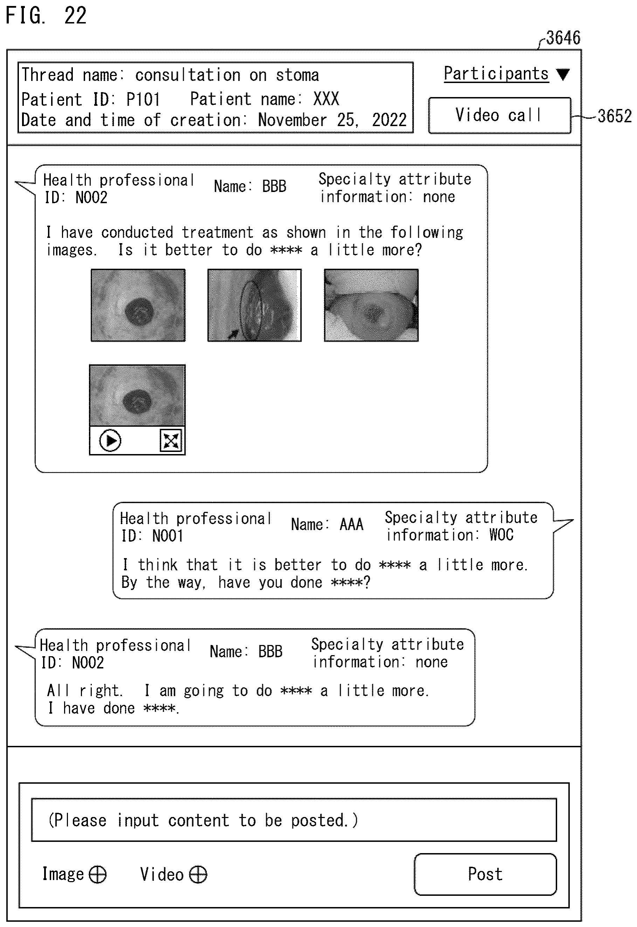

An operation as a trigger for executing a video call may be accepted in, for example, the thread screen 3646. FIG. 22 shows an example of a thread screen 3646 according to the present modification. As shown in FIG. 22, the thread screen 3646 according to the present modification includes a button 3652. The button 3652 accepts an operation for executing a video call. When accepting the operation through the button 3652, the information exchange unit 364 calls, for example, a video call application to execute a video call.

The request destination of the video call may be each of all the health professionals in the group or may be one of said health professionals. The flow of a process in a case where a video call request is made with respect to all the health professionals in the group will be described. The processor 35 follows an instruction from the information exchange unit 364 to transmit, to the server 2, a video call request including the thread ID of the thread displayed in the thread screen 3646 in which the operation has been accepted through the button 3652. The server 2 searches the group information database 223 so as to obtain health professional IDs associated with a group ID corresponding to the thread ID included in the video call request. Subsequently, the server 2 searches the account information database 221 so as to obtain communication addresses with which the health professional IDs obtained from the group information database 223 are associated. Subsequently, the server 2 transmits the video call request to terminals 3 excluding the video call request source among the terminals 3 having the obtained respective communication addresses. When any of the terminals 3 having received the video call request accepts, from the corresponding health professional, an operation for taking the call, a video call between said terminal 3 and the terminal 3 which is the video call request source is started. The information exchange unit 364 of each of the terminals 3 in which the video call has been started causes the output device 33 thereof to display a video call screen 3653.

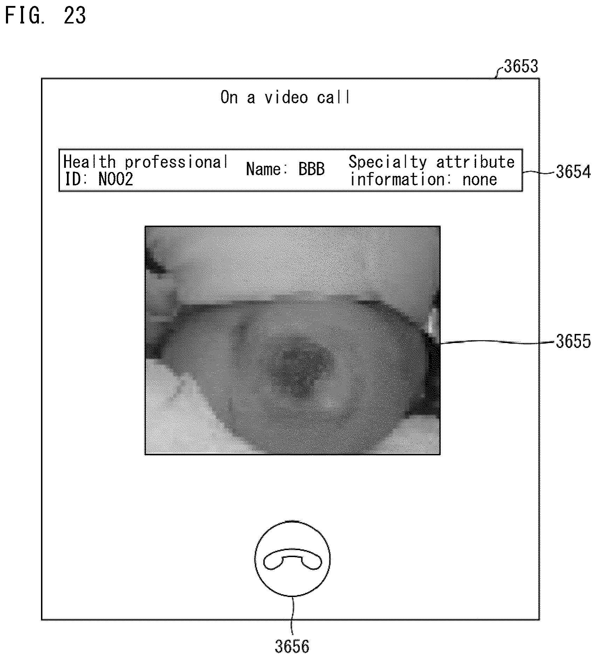

FIG. 23 shows an example of the video call screen 3653. As shown in FIG. 23, the video call screen 3653 includes, for example, fields 3654 and 3655 and a button 3656. In the field 3654, information about the health professional who is the other party of the video call is displayed. In the field 3655, a moving image being taken with the terminal 3 of the health professional who is the other party of the video call is displayed. In an example shown in FIG. 23, a moving image showing a lesion of the patient is displayed in the field 3655. When accepting an operation through the button 3656, the processor 35 follows an instruction from the information exchange unit 364 to end the video call. The information system 1 may have a function of allowing a voice call among the health professionals in the group.

### [Modification 2: Video Call Reservation]

The information system 1 may have a function of reserving a video call to be performed among the health professionals. The reservation function is useful for making a video call with a health professional for whom it is difficult to immediately respond to a video call request.

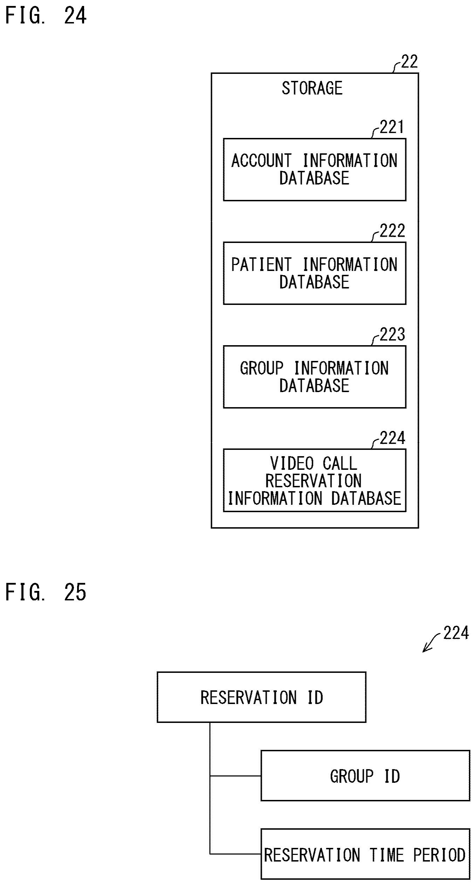

A server 2 according to the present modification has a function of managing a reservation for a video call. As shown in FIG. 24, a storage 22 of the server 2 according to the present modification further stores therein a video call reservation information database 224 in addition to the account information database 221, the patient information database 222, and the group information database 223. The video call reservation information database 224 is a database for storing therein video call reservation information and is, for example, an RDB. FIG. 25 shows an example of a data structure in a case where the video call reservation information database 224 is an RDB. As shown in FIG. 25, the video call reservation information includes, for example, a reservation ID, a group ID, and a reservation time period.

The reservation ID is identification information for a reservation. As shown in FIG. 25, for example, the group ID and the reservation time period are associated with the reservation ID. Another piece of attribute information about the reservation (e.g., a reservation name, the person who has made the reservation, and the like) may be associated with the reservation ID. The group ID is a group ID of the group for executing the reserved video call identified by the reservation ID and is based on the same coding scheme as that of the group ID managed in the group information database 223. The reservation time period is a time period for executing the reserved video call identified by the reservation ID.

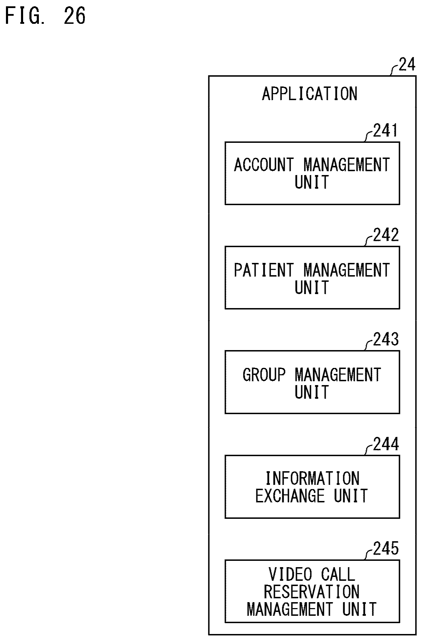

As shown in FIG. 26, an application 24 of the server 2 according to the present modification further includes a video call reservation management unit 245 in addition to the account management unit 241, the patient management unit 242, the group management unit 243, and the information exchange unit 244. The processor 23 follows an instruction from the video call reservation management unit 245 to: receive a video call reservation request transmitted from any of the terminals 3 according to an input operation performed by the corresponding health professional; and store, in the video call reservation information database 224, video call reservation information included in the video call reservation request.

In addition, the processor 23 follows an instruction from the video call reservation management unit 245 to: receive a video call reservation search request transmitted from the terminal 3 according to an input operation performed by the health professional; upon the reception, search the video call reservation information database 224 so as to obtain reservation information indicated by a reservation ID associated with a group ID corresponding to a thread ID included in the video call reservation search request; and transmit the obtained reservation information to the terminal 3 which is the video call reservation search request source.

In addition, the processor 23 may follow an instruction from the video call reservation management unit 245 to transmit, to the terminal 3, notification information indicating that the time point of starting the reserved video call is approaching. In this case, the processor 23 follows an instruction from the video call reservation management unit 245 to: search the video call reservation information database 224; and check presence/absence of reservation information with which a reservation time period starting after a predetermined time (e.g., 15 minutes) elapses from the present time point is associated. In a case where the reservation information is present, the processor 23 follows an instruction from the video call reservation management unit 245 to search the group information database 223 so as to obtain health professional IDs associated with the group ID included in the reservation information. Subsequently, the processor 23 follows an instruction from the video call reservation management unit 245 to search the account information database 221 so as to obtain communication addresses associated with the respective health professional IDs obtained from the group information database 223. Subsequently, the processor 23 follows an instruction from the video call reservation management unit 245 to transmit, to each of the terminals 3 having the obtained respective communication addresses, the notification information indicating that the time point of starting the video call is approaching. The terminal 3 having received the notification information causes the output device 33 thereof to display the notification information. The notification information is given in the form of, for example, a pop-up notification, a banner notification, or the like.

An information exchange unit 364 of the terminal 3 according to the present modification has a video call reservation function and a video call reservation search function. Firstly, the video call reservation function will be described.

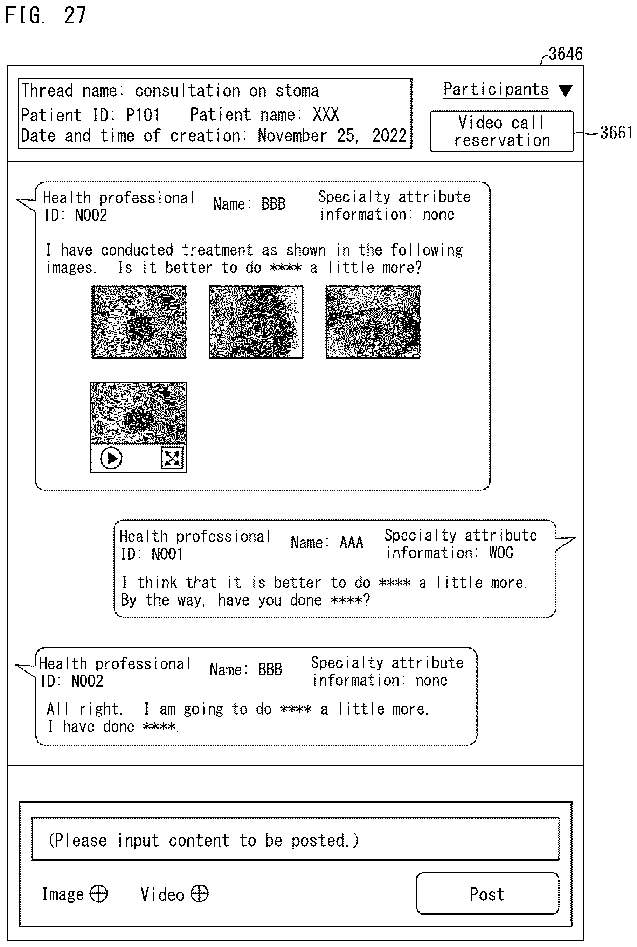

An operation for starting a video call reservation is accepted in, for example, the thread screen 3646. FIG. 27 shows an example of a thread screen 3646 according to the present modification. As shown in FIG. 27, the thread screen 3646 according to the present modification includes a button 3661. When accepting an operation through the button 3661, the processor 35 follows an instruction from the information exchange unit 364 to create a video call reservation screen 3657 and causes the output device 33 to display the video call reservation screen 3657.

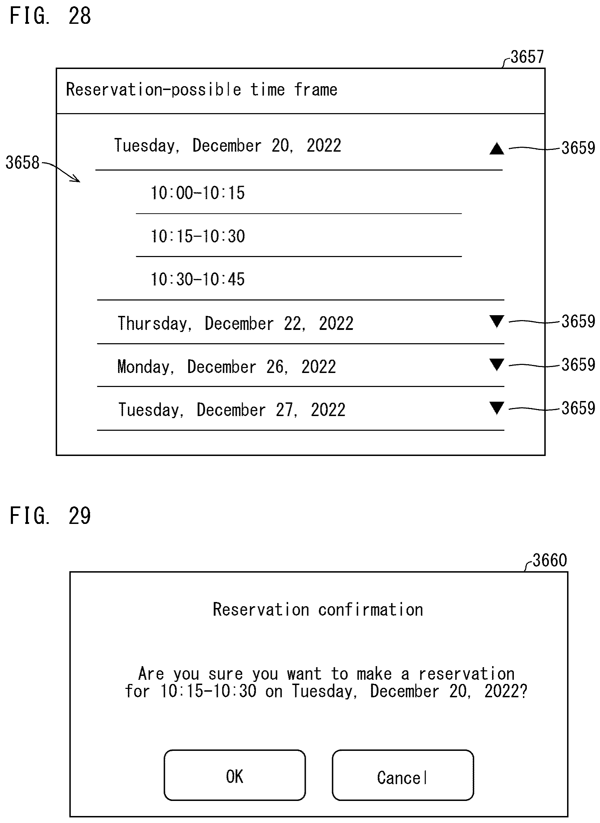

The video call reservation screen 3657 is a screen for accepting a reservation for a video call. FIG. 28 shows an example of the video call reservation screen 3657. As shown in FIG. 28, the video call reservation screen 3657 includes, for example, lists 3658 and buttons 3659. In each of the lists 3658, reservation-possible time frames for a video call are displayed, and selection and input of a desired reservation time frame are accepted. The reservation-possible time frames only have to be set in consideration of work schedules, of the health professionals in the group, obtained from a schedule management system in the medical institutions to which the health professionals belong, for example. Each of the buttons 3659 is a toggle button for accepting an operation for switching between displaying and hiding of the reservation-possible time frames.

When accepting selection and input of a desired reservation time frame in any of the lists 3658, the processor 35 may follow an instruction from the information exchange unit 364 to: create a confirmation screen 3660 shown in FIG. 29; and cause the output device 33 to display the confirmation screen 3660. When the acceptance of the reservation time frame is confirmed, the processor 35 follows an instruction from the information exchange unit 364 to transmit a video call reservation request including video call reservation information to the server 2. The reservation information includes: a newly created reservation ID; the reservation time frame accepted in the list 3658; and the thread ID of the thread displayed in the thread screen 3646 which is the calling source of the video call reservation screen 3657.

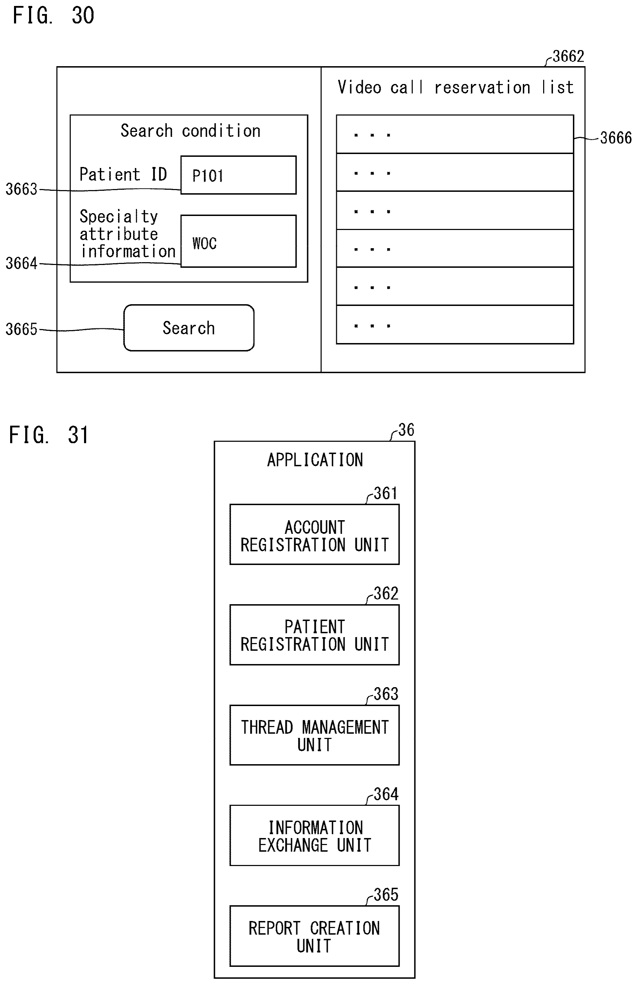

Next, the video call reservation search function will be described. The processor 35 follows an instruction from the information exchange unit 364 to: create a video call reservation search screen 3662 according to an input operation performed by the health professional; and cause the output device 33 to display the video call reservation search screen 3662. The video call reservation search screen 3662 is a screen for accepting a search for a desired piece of reservation information. FIG. 30 shows an example of the video call reservation search screen 3662. As shown in FIG. 30, the video call reservation search screen 3662 includes, for example, fields 3663 and 3664, a button 3665, and a list 3666.

In the fields 3663 and 3664, input of conditions for searching for a desired reservation is accepted. In the field 3663, input of a patient ID is accepted. In the field 3664, input of a piece of specialty attribute information is accepted. The field 3664 is preferably of a selection style. Any of pieces of specialty attribute information displayed in a drop-down list prepared in advance or the like is preferably selected to automatically set, in the field 3664, the piece of specialty attribute information.

When accepting an operation through the button 3665, the processor 35 follows an instruction from the information exchange unit 364 to search the thread information database 341 so as to obtain a thread ID associated with the patient ID and the specialty attribute information inputted in the fields 3663 and 3664. Then, the information exchange unit 364 causes transmission of a video call reservation search request including the obtained thread ID to the server 2 and causes pieces of reservation information, which have been given as replies from the server 2 in response to the transmission, to be listed in the list 3666.

When accepting an operation of selecting a piece of reservation information in the list 3666, the processor 35 follows an instruction from the information exchange unit 364 to call the video call application and execute a video call when the start of the reservation time period associated with the selected piece of reservation information arrives, for example.

### [Modification 3: Report Creation]

The information system 1 may enable creation of a report including a message having been subjected to information exchange in a thread. Consequently, burden on a health professional taken for the report creation can be mitigated.

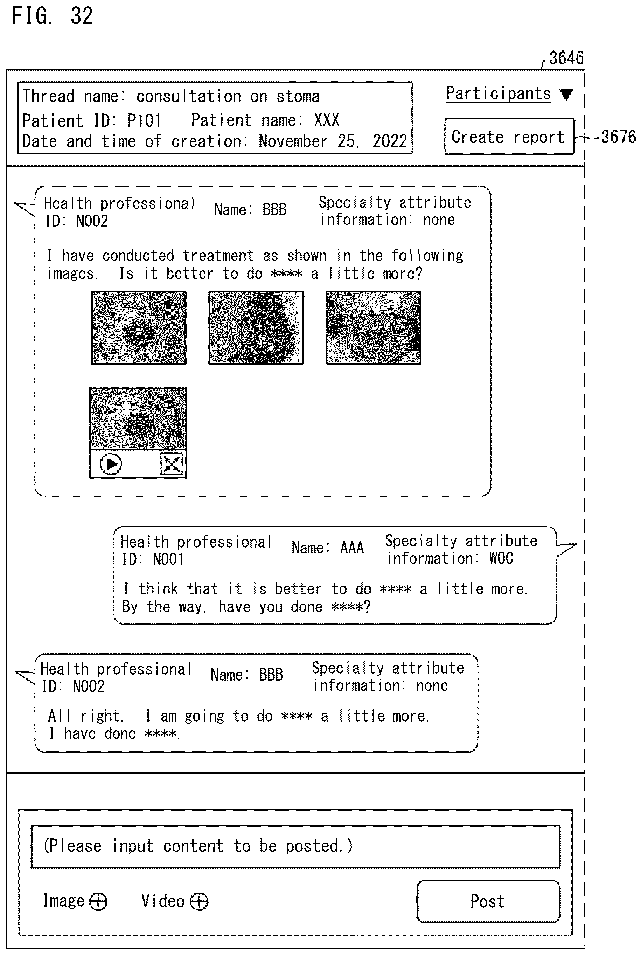

An application 36 of each of terminals 3 according to the present modification further includes, as shown in FIG. 31, a report creation unit 365 in addition to the account registration unit 361, the patient registration unit 362, the thread management unit 363, and the information exchange unit 364. An operation for starting report creation is accepted in, for example, the thread screen 3646. FIG. 32 shows an example of a thread screen 3646 according to the present modification. As shown in FIG. 32, the thread screen 3646 according to the present modification includes a button 3676. When accepting an operation through the button 3676, the processor 35 follows an instruction from the report creation unit 365 to: create a report creation screen 3671; and cause the output device 33 to display the report creation screen 3671.

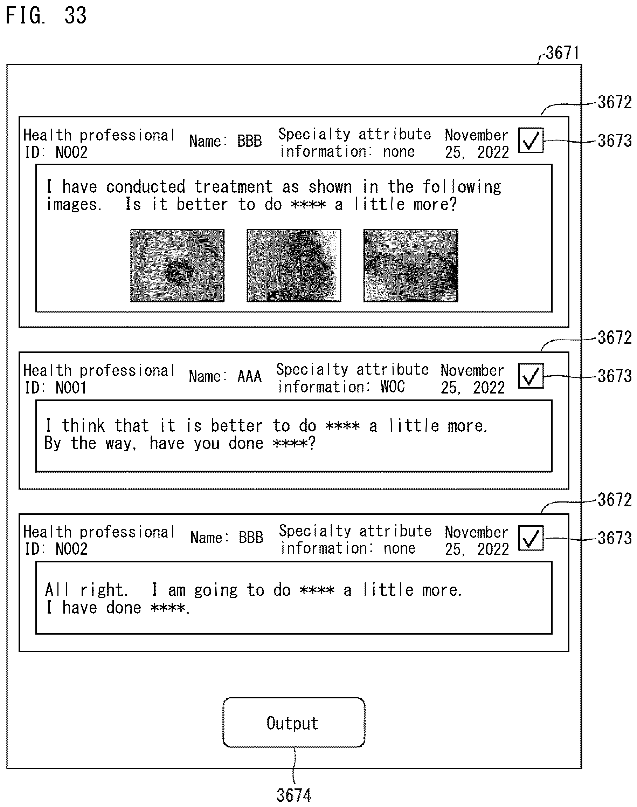

The report creation screen 3671 is a screen for creating a report from a message having been subjected to information exchange in the thread. FIG. 33 shows an example of the report creation screen 3671. As shown in FIG. 33, the report creation screen 3671 includes, for example, fields 3672, checkboxes 3673, and a button 3674. In each of the fields 3672, a message is displayed. In each of the checkboxes 3673, selection as to whether or not to output the corresponding message in a report is accepted.

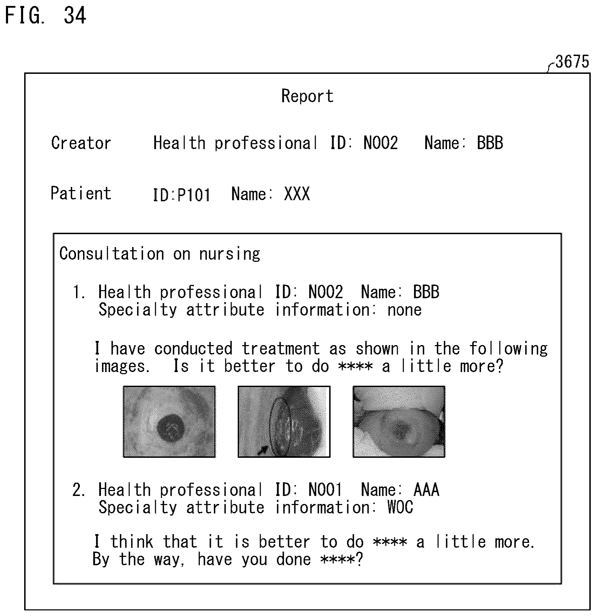

When accepting an operation through the button 3674, the processor 35 follows an instruction from the report creation unit 365 to cause the output device 33 to output a report 3675 including the messages selected by using the checkboxes 3673. FIG. 34 shows an example of the report 3675.

### [Modification 4: Patient Information]

The patient information database 222 may further store therein, in association with the patient ID, specialty attribute information indicating a specialty in medicine to be provided to the patient identified by the patient ID. In this case, the patient registration screen 3621 shown in FIG. 17 may be further provided with a field for accepting input of the specialty attribute information. In this case, when a creation request is made in a state where a patient ID is inputted and no specialty attribute information is inputted in the creation request screen 3631 shown in FIG. 18, the processor 23 may follow an instruction from the group management unit 243 to: obtain, from the patient information database 222, specialty attribute information associated with the patient ID included in the creation request; obtain, from the account information database 221, health professionals with whom the obtained specialty attribute information is associated; and create a group including the obtained health professionals.

### [Modification 5: Group]

The group X1 created by the information system 1 includes: the general nurse G2 and the home-visiting nurse V2 in charge of the patient A; and the specialized nurses S1 and S2. Alternatively, the group X1 may be a group composed of the specialized nurses without including the general nurse G2 and the home-visiting nurse V2.

The present invention is not limited to the embodiments described above, and various modifications can be made without departing from the scope of the claims. Embodiments obtained by combining as appropriate technological means disclosed in different embodiments are also included in the technological scope of the present invention.

### DESCRIPTION OF THE REFERENCE CHARACTERS

1 information system
2 server (information processing device)
3 terminal
22, 34 storage
23, 35 processor (controller)
24, 36 application
3675 report
M1, M2 medical institution
S1, S2, S3, S4 specialized nurse (health professional)
G1, G2 general nurse (health professional)
V1, V2 home-visiting nurse (health professional)
X1, X2, X3 group

## Claims

1. An information exchange method for exchanging information about a patient among a plurality of health professionals by an information system, the information exchange method comprising:
receiving a group creation request based on at least one of patient identification information for identifying the patient and specialty attribute information about a specialty, in medicine, that is necessary for providing medicine to the patient;
searching for a health professional satisfying the group creation request;
creating, according to a result of the searching, a group including a health professional having made the group creation request and at least one of a health professional in charge of the patient and a health professional having the specialty, in medicine, that is necessary for providing medicine to the patient; and
transmitting information about the patient transmitted from a terminal of one of the health professionals in the created group, the information being transmitted to a terminal of another one of the health professionals in the group.

2. The information exchange method of claim 1, wherein
the searching includes searching for, as the health professional satisfying the group creation request, each of the health professional in charge of the patient and the health professional having the specialty in medicine, and
the creating of the group includes creating a group including the health professional in charge of the patient, the health professional having the specialty in medicine, and the health professional having made the group creation request.

3. The information exchange method of claim 2, wherein the group creation request is based on the patient identification information and the specialty attribute information.

4. The information exchange method of any one of claims 1 to 3, wherein the searching includes searching a database, in which each of pieces of health professional identification information for identifying health professionals and at least one of a corresponding piece of the patient identification information and a corresponding piece of the specialty attribute information are stored in association with each other, for the health professional satisfying the group creation request.

5. The information exchange method of claim 4, wherein
the group creation request is based on the patient identification information, and
each of the pieces of the patient identification information and the corresponding piece of the specialty attribute information are stored in association with each other in the database.

6. The information exchange method of claim 1, wherein the group is a group including a plurality of health professionals as at least one of the health professional in charge of the patient and the health professional having the specialty in medicine.

7. The information exchange method of claim 1, wherein the health professional having the specialty in medicine is a person belonging to a medical institution associated with the patient.

8. The information exchange method of claim 1, wherein the receiving of the group creation request includes receiving the group creation request from a terminal of the health professional having made the group creation request.

9. The information exchange method of claim 1, wherein the group is created for each of a plurality of patients regarding whom information exchange is to be performed by the information system.

10. The information exchange method of claim 1, wherein
a plurality of the group creation requests different from one another are received for the patient who is identical, and
the group is created for each of the plurality of the group creation requests.

11. The information exchange method of claim 10, wherein a group including all health professionals included in at least one of a plurality of the groups created for the patient who is identical is created.

12. The information exchange method of claim 1, further comprising
storing each of pieces of health professional identification information for identifying health professionals and at least one of a corresponding piece of the patient identification information and a corresponding piece of the specialty attribute information in association with each other.

13. The information exchange method of claim 1, further comprising
creating a report including the information about the patient transmitted from the terminal of the one health professional in the group.

14. The information exchange method of claim 1, wherein the information about the patient transmitted from the terminal of the one health professional in the group is at least one of text data, still image data, and moving image data.

15. The information exchange method of claim 1, further comprising
executing, according to a call request transmitted from the terminal of the one health professional in the group, a voice call or a video call between the terminal of the one health professional and the terminal of the other health professional.

16. The information exchange method of claim 1, wherein the health professional having the specialty in medicine is a person having a specialist qualification regarding medicine.

17. The information exchange method of claim 1, wherein the specialty attribute information includes information indicating a specialty in nursing.

18. The information exchange method of claim 1, wherein
the specialty attribute information is information indicating a type of nursing, and
the group is created for each type of nursing to be performed on the patient.

19. The information exchange method of claim 1, wherein the group includes a plurality of health professionals belonging to medical institutions different from one another.

20. A program for causing a computer to function as an information processing device included in an information system configured to exchange information about a patient among a plurality of health professionals, the information processing device being configured to:
receive a group creation request based on at least one of patient identification information for identifying the patient and specialty attribute information about a specialty, in medicine, that is necessary for providing medicine to the patient;
search for a health professional satisfying the group creation request;
create, according to a result of the searching, a group including a health professional having made the group creation request and at least one of a health professional in charge of the patient and a health professional having the specialty, in medicine, that is necessary for providing medicine to the patient; and
transmit information about the patient transmitted from a terminal of one of the health professionals in the created group, the information being transmitted to a terminal of another one of the health professionals in the group.

21. An information system configured to exchange information about a patient among a plurality of health professionals, the information system comprising:
an information processing device; and
a terminal of each of the health professionals, wherein
the information processing device
receives, from the terminal of any of the health professionals, a group creation request based on at least one of patient identification information for identifying the patient and specialty attribute information about a specialty, in medicine, that is necessary for providing medicine to the patient,
searches for a health professional satisfying the group creation request,
creates, according to a result of the searching, a group including a health professional having made the group creation request and at least one of a health professional in charge of the patient and a health professional having the specialty, in medicine, that is necessary for providing medicine to the patient, and
transmits information about the patient transmitted from the terminal of one of the health professionals in the created group, the information being transmitted to the terminal of another one of the health professionals in the group.

22. A program for causing a computer to function as a terminal of each of a plurality of health professionals utilizing an information system configured to exchange information about a patient among the health professionals, the terminal being configured to:
transmit, to an information processing device included in the information system, a group creation request based on at least one of patient identification information for identifying the patient and specialty attribute information about a specialty, in medicine, that is necessary for providing medicine to the patient;
accept input of information about the patient; and
transmit and receive the information about the patient among the terminals of health professionals in a group created on the basis of the group creation request.
